# FASCICULE DE BREVET EUROPEEN

(11) **EP 0 577 705 B1**
(45) Date de publication et mention de la délivrance du brevet: **05.06.1996**
(21) Numéro de dépôt: 92908261.8
(22) Date de dépôt: 26.03.1992
(51) Int. Cl.: C07K 5/06, C12P 21/04

(54) **PROCEDE DE PREPARATION ENZYMATIQUE DE MACROLACTONE**
VERFAHREN ZUR ENZYMATISCHEN HERSTELLUNG VON MAKROLAKTON
METHOD OF ENZYMATIC PREPARATION OF MACROLACTONE

(30) Priorité: 28.03.1991 FR 9103779
(43) Date de publication de la demande: 12.01.1994
(73) Titulaire: RHONE-POULENC RORER S.A., 92160 Antony (FR)
(72) Inventeur: BLANCHE, Francis, F-75019 Paris (FR); THIBAUT, Denis, F-75013 Paris (FR)
(74) Mandataire: Savina, Jacques
(86) Numéro de dépôt international: FR9200270
(87) Numéro de publication internationale: WO9217491

(56) Documents cités:
- JOURNAL OF THE AMERICAN CHEMICAL SOCIETY. vol. 111, no. 15, 19 Juillet 1989, GASTON, PA US pages 5931 - 5935; M B PURVIS ET AL.: 'Biosynthesis of antibiotics of the virginiamycin family. 8. formation of the dehydroproline residue' cité dans la demande

## Description

La présente invention concerne le domaine des bioconversions. Plus particulièrement, elle concerne un procédé enzymatique de préparation de macrolactones de formule (1) à partir de macrolactone de formule (2) au moyen d'un microorganisme ou d'une préparation acellulaire issue de ce microorganisme.

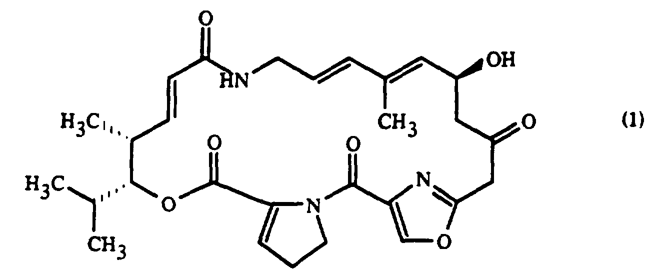

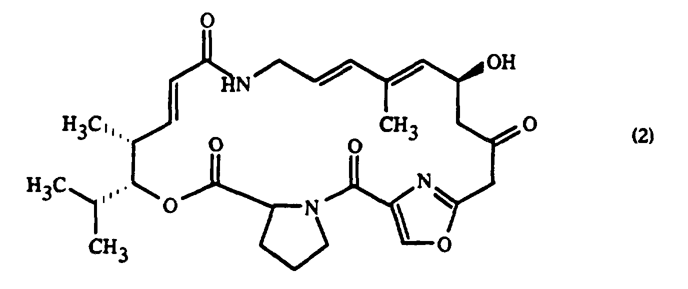

Les macrolactones de formule (1) et (2) sont connues sous différents noms en fonction de leurs origines (tableau).

| Formule (1) | Formule (2) |
|---|---|
| pristinamycine PIIA | pristinamycine IIB |
| mikamycine A | |
| ostreogrycine A | ostreogrycine G |
| streptogramine A | |
| synergistine A1 | |
| vernamycine A | |
| virginiamycine M1 | virginiamycine M2 |

Ces molécules présentent la structure générale des macrolactones polyinsaturées que l'on rencontre dans les antibiotiques de la famille des streptogramines. Ces antibiotiques sont en effet constitués de l'association synergique d'une macrolactone polyinsaturée (composante du groupe A) et d'un depsipeptide (composante du groupe B). Le mode d'action et l'activité antimicrobienne de ces antibiotiques ont été étudiés par différents auteurs (Tanaka et al. Antibiotics vol.III p. 487, Springer Berlin, 1975 ; Vasquez et al. Antibiotics vol.III p. 521, Springer Berlin, 1975).

Il ressort notamment de ces différentes études qu'une meilleure synergie est obtenue lorsque l'on utilise uniquement la macrolactone (1) comme composante du groupe A.

Par ailleurs, une caractéristique des macrolactones polyinsaturées du groupe A des streptogramines est d'être peu solubles en milieu aqueux. Ceci constitue un inconvénient important dans le développement pharmacologique de ces molécules, puisque leurs modes d'administration en sont très limités.

Afin de pallier à ce problème, une nouvelle génération de dérivés hémisynthétiques hydrosolubles a été mise au point (EP 135410 ; EP 191662 ; US 4775753). La voie d'hémisynthèse utilisée consiste essentiellement en l'addition de thiol sur la liaison insaturée en 2-3 de la déhydroproline des macrolactones. Toutefois, compte tenu de la structure des macrolactones, seule la macrolactone (1) peut être utilisée dans cette voie d'hémisynthèse.

Or, dans les systèmes de production actuels, les macrolactones de formule (1) et (2) sont synthétisées simultanément, et en mélange avec d'autres composantes des streptogramines.

Il est donc important de pouvoir augmenter la proportion de composé (1) par rapport au composé (2) dans les milieux de production.

Il est également important de pouvoir valoriser les composés (2) qui sont co-synthétisés avec le composé (1), et isolés du moût de fermentation de manière parallèle.

Récemment, Purvis et al ont évoqué la possibilité que la macrolactone (2) soit un intermédiaire de biosynthèse de la macrolactone (1) (J. Am. Chem. Soc., 1989, 111, 5931). Rien cependant dans ce document ne décrit ni ne donne les moyens d'exploiter ce mécanisme réactionnel.

La demanderesse a maintenant montré qu'il est possible de convertir, à des taux élevés, la macrolactone (2) en macrolactone (1) au moyen d'un microorganisme ou d'une préparation acellulaire issue de celui-ci.

Un objet de la présente invention réside donc dans un procédé de préparation d'un composé de formule (1) caractérisé en ce que l'on traite un composé de formule (2) en présence d'un microorganisme ou d'une préparation acellulaire issue de ce microorganisme, capable d'oxyder la liaison 2-3 de la D-proline des macrolactones polyinsaturées du groupe A des streptogramines en déhydroproline.

La présente invention peut être utilisée in vitro comme in vivo.

In vitro, elle permet de synthétiser la macrolactone (1) à partir de macrolactone (2) isolée du moût de fermentation, qui n'est pas directement valorisable. L'invention permet ainsi de convertir à des taux élevés la macrolactone (2) en substrat pour des réactions d'hémisynthèse.

In vivo, elle permet d'augmenter le taux de macrolactone (1) au détriment de la macrolactone (2) directement dans les mélanges issus de la fermentation. Il est en effet possible d'introduire directement dans le milieu de production des macrolactones, un microorganisme ou une préparation acellulaire issue de celui-ci et dotée de l'activité d'oxydation.

Dans un mode particulier de mise en oeuvre du procédé de l'invention, le microorganisme ou la préparation acellulaire qui en est issue est ajouté directement dans le milieu de production de la macrolactone (2).

Dans un mode préféré de mise en oeuvre de l'invention, on traite le composé (2) au moyen d'une préparation acellulaire.

La présente invention permet ainsi d'obtenir de manière simple la macrolactone (1) avec des taux de conversion du composé (2) atteignant 50 % en utilisant les microorganismes entiers, et dépassant 90 % lorsque des préparations acellulaires issues de ces microorganismes sont utilisées.

Les microorganismes utilisables dans la présente invention peuvent être sélectionnés de plusieurs manières. Notamment, la présente invention décrit un test permettant d'identifier les microorganismes possédant l'activité enzymatique requise, et de donner une estimation quantitative de cette activité. Ce test consiste à effectuer les étapes suivantes :
- incuber une culture du microorganisme étudié ou une préparation acellulaire issue de celui-ci en présence du composé (2) marqué,
- prélever au cours du temps des échantillons du milieu de culture,
- séparer les composés (1) et (2),
- mesurer le marquage incorporé dans le composé (1), et
- déterminer le rapport de ce marquage sur le marquage introduit.

Le rapport ainsi obtenu permet d'accéder au taux de conversion en composé (1), et donc au niveau d'activité enzymatique du microorganisme en culture. Il est entendu que la sélection des microorganismes peut être effectuée par toute autre méthode permettant de révéler la présence de l'activité enzymatique, et que l'approche quantitative est facultative.

En utilisant ce mode de sélection, différents microorganismes ont été mis en évidence, dotés d'une activité enzymatique élevée.

Avantageusement, dans le procédé de l'invention, le microorganisme utilisé est choisi parmi les actinomycètes et les champignons.

En particulier, les microorganismes producteurs de streptogramines conviennent généralement dans le procédé de l'invention. Il en est de même pour les microorganismes producteurs de la macrolactone (1).

Parmi ceux-ci, on peut citer plus particulièrement les souches suivantes:
Streptomyces pristinaespiralis
Streptomyces alborectus
Streptomyces diastaticus
Streptomyces graminofaciens
Streptomyces mitakaensis
Streptomyces loïdensis
Streptomyces olivaceus
Streptomyces ostréogriseus
Streptomyces virginiae, ou
Micromonospora.

Ces microorganismes peuvent être cultivés dans des conditions standard de fermentation aérobie. Notamment, le milieu nutritif est généralement constitué d'une source de carbone, d'une source d'azote et de sels minéraux. Comme source de carbone on peut utiliser en particulier sucres, huiles, acides organiques, dextrines, amidons, glycérol... Comme source d'azote, on peut citer les acides aminés, farines végétales et extraits (malte, soja, graines de coton, tomates, maïs...), viscères, hydrolysats variés (caséine, levure...) et sous-produits industriels tels que "distillers soluble". Comme source minérales on peut utiliser des chlorures, nitrates, carbonates, sulfates et phosphates de sodium, potassium, ammonium ou calcium, ou des éléments trace tels que magnésium, fer, cuivre, zinc, manganèse ou cobalt.

De plus, en utilisant le test de sélection décrit ci-avant, la demanderesse a montré que l'activité enzymatique recherchée était exprimée de manière transitoire par les différents microorganismes étudiés. Des cinétiques d'expression de cette activité enzymatique ont été réalisées, qui permettent de définir, pour les microorganismes étudiés, la période de croissance au cours de laquelle l'expression de l'activité enzymatique est optimale. Ceci permet donc d'améliorer considérablement les rendements du procédé de l'invention.

Dans un mode préféré de l'invention, on utilise un microorganisme ou une préparation acellulaire issue d'un microorganisme en phase de production optimale de l'activité enzymatique.

Toujours selon la présente invention, il est possible, après une première étape de sélection, d'utiliser les microorganismes possédant l'activité enzymatique requise pour préparer des souches dérivées, présentant de meilleures potentialités catalytiques, et/ou permettant une exploitation industrielle plus performante. De telles souches peuvent être obtenues en utilisant différents outils de mutagénèse, tels que notamment:
- des agents physiques : rayons X, rayons ultra-violet,
- des agents chimiques : agents alkylants (Ethylméthanesulfonate, nitrosoguanidine, NQO...), bialkylants ou intercalants,
- des systèmes d'insertion mutationnel sur l'ADN : transposons, plasmides intégratifs, phages ou prophages...
   ou toute autre technique connue de l'homme de l'art.

Encore selon la présente invention, il est possible de sélectionner au sein d'une population de cellules présentant l'activité enzymatique requise, celles qui possèdent la meilleur activité enzymatique et/ou donnent les meilleurs rendements dans le procédé de l'invention.

Dans un mode particulier de mise en oeuvre du procédé de l'invention, on utilise un microorganisme producteur de streptogramines ou dérivé d'un microorganisme producteur de streptogramines.

Préférentiellemnt, on utilise un microorganisme choisi parmi le groupe comprenant :
S.pristinaespiralis ATCC 25486
S.ostreogriseus ATCC 27455
S.mitakaensis ATCC 15297
S.olivaceus ATCC 12019
S.loïdensis ATCC 11415
et les microorganismes dérivés de ceux-ci.

La réaction enzymatique de conversion peut être effectuée soit dans le milieu de fermentation, soit dans un milieu aqueux tamponné, selon que l'on utilise un microorganisme intact ou une préparation acellulaire, et que l'on opère directement dans le milieu de production des macrolactones ou avec un composé (2) isolé du moût de fermentation.

Lorsqu'une préparation acellulaire est utilisée, il peut être avantageux d'ajouter au milieu réactionnel un ou plusieurs cofacteurs enzymatiques. Notamment, les cofacteurs suivants permettent d'améliorer les rendements de la réaction : NAD, NADP, NADH, NADPH, FAD et FMN.

Les autres paramètres de la réaction sont adaptés par l'homme de l'art en fonction des conditions utilisées.

Un autre objet de l'invention concerne les préparations acellulaires possédant une activité enzymatique capable d'oxyder la liaison 2-3 de la D-proline des macrolactones polyinsaturées du groupe A des streptogramines en déhydroproline.

Ces préparations acellulaires peuvent être obtenues par différentes méthodes. En particulier, elles peuvent être obtenues par (a) rupture des microorganismes décrits ci-avant et (b) élimination éventuelle des débris cellulaires.

La rupture des microorganismes peut être effectuée par différentes techniques, et notamment des techniques physiques, chimiques ou enzymatiques. A titre d'exemple, on peut citer comme techniques physiques l'ultrasonification, le broyage aux billes de verre ou la "French press" et comme technique enzymatique la lyse au lysozyme.

La rupture peut être réalisée directement dans le milieu de fermentation des cellules. Toutefois, on préfère opérer en milieu aqueux tamponné. A cet égard, il est possible d'utiliser un tampon inorganique (phosphate de potassium, ...) ou organique (Bis-tris, Bis-tris propane). Par ailleurs, la réaction est avantageusement réalisée en présence d'un agent réducteur. On peut notamment utiliser le dithiothréitol. Selon le microorganisme de départ et les conditions de la rupture, le pH de la préparation acellulaire est ajusté entre pH 5,0 et pH 8,0, avec une préférence entre 6 et 7.

Lorsque cela est désiré, les débris cellulaires peuvent être éliminés de diverses façons, la plus simple consistant à centrifuger la suspension obtenue après rupture et à reprendre le surnageant.

Pour verifier que la préparation acellulaire possède bien l'activité enzymatique recherchée, ou pour sélectionner la préparation acellulaire la plus active, il est possible d'utiliser le test précédemment décrit pour la sélection des microorganismes.

Par ailleurs, pour obtenir des préparations acellulaires ayant une activité optimale, il est possible, avant de procéder à l'étape de rupture, de déterminer la phase de croissance des microorganismes entiers correspondant à l'expression maximale de l'activité enzymatique. On peut en particulier utiliser les courbes cinétiques obtenues avec le test décrit précédemment.

Préférentiellement, dans le procédé d'obtention des préparations acellulaires de l'invention, l'étape de rupture est réalisée sur des microorganismes en phase de production optimale de l'activité enzymatique.

D'autres objets et avantages de la présente invention apparaîtront à la lecture des exemples qui suivent, qui doivent être considérés comme illustratifs et non limitatifs.

### Exemple 1

Cet exemple décrit la préparation de macrolactone (2) radiomarquée au carbone 14.

750 µCi de [U-¹⁴C]-L-Proline sont ajoutés dans une culture en erlenmeyer de S.pristinaespiralis sur milieu de production (cf. exemple 3) âgée de 17 heures. Quand la culture est âgée de 24 heures, elle est recueillie et les macrolactones (1) et (2) sont extraites selon le protocole suivant :
55 ml de tampon phosphate 0,1M pH 2,9 et 25 ml d'acétonitrile sont ajoutés aux 30 ml de moût, et le mélange est agité puis centrifugé. Après évaporation partielle du surnageant, celui-ci est extrait 3 fois par un volume de dichlorométhane, et les 3 phases chlorométhyléniques sont regroupées et mises à sec. Le résidu est repris par du chloroforme à 2 % de méthanol puis injecté sur une colonne de silice nue et le composé (2) est élué dans du chloroforme à 5 % de méthanol. Le composé (2) est purifié par chromatographie sur couche mince de silice nue avec pour éluant du chloroforme à 8 % de méthanol. Juste avant usage, il est repurifié par CLHP sur colonne Nucléosil C8 (système décrit dans l'exemple 3).

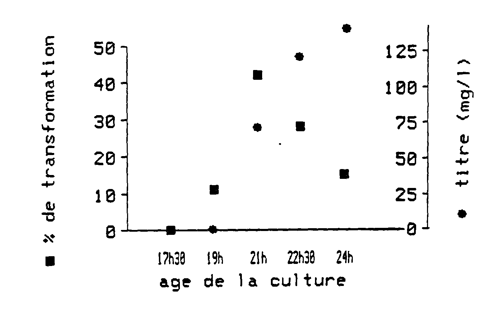

Comme pour les préparations acellulaires (cf. figure 1), le moment choisi pour ajouter la proline marquée est important pour obtenir des activités spécifiques élevées.

### Exemple 2

Cet exemple décrit un test permettant la sélection des microorganismes utilisables dans l'invention. Il illustre également comment le niveau d'activité enzymatique est dépendant du moment du prélèvement des cultures par rapport au début de la production des Streptogramines.

Le microorganisme testé est une culture de S.olivaceus ATCC 12019 cultivée dans les conditions de l'exemple 6. A différents temps, une préparation acellulaire est préparée à partir d'échantillons de cette culture, de la manière suivante : 5 g d'un culot de centrifugation, lavé par un tampon phosphate 0,1M pH 7,2 à 10 % v/v de glycérol, sont repris par 10 ml de tampon Bis tris propane 100 mM pH 6,8 contenant 5 mM de dithiothréitol et 0,2 mg/l de lysozyme. La suspension est incubée 30 minutes à 27°C, puis centrifugée à 30000 g pendant 30 minutes.

Un extrait acellulaire contenant 50 µg de protéines (issu de la préparation obtenue ci-dessus) est incubé dans un volume total de 500 µl dans du tampon Bis-tris propane 50 mM pH 6,8 contenant 0,25 µmole de NADH ; 1 nmole de FMN et 3,65 µg de composé (2) marqué au carbone 14 selon l'exemple 1 (soit 55nCi) pendant 1 heure à 27°C. La réaction est stoppée par addition de 500 µl d'acétonitrile et 500 µl d'acide chlorhydrique 0,1N.

Après homogénéisation et centrifugation, 200 µl du surnageant sont injectés sur une colonne analytique nucléosil C8 5µ de 15 cm éluée à 0,8 ml/mn par un mélange de 34 % CH3CN et 66 % tampon phosphate 0,1M pH 2,9. Les composés (1) et (2) sont dosés par détection photométrique à 206 nm et par détection radiochimique.

Les résultats obtenus sont présentés sur la figure 1. Ils indiquent que S.olivaceus possède l'activité enzymatique recherchée. Ils montrent également que cette activité est exprimée de manière transitoire, la phase de production optimale se situant autour de 21 heures de culture. Ils montrent enfin que l'activité apparaît au début de la phase production de Streptogramines mais que des extraits acellulaires actifs peuvent toutefois être préparés avant le démarrage de la production de Streptogramines.

### Exemple 3

Cet exemple illustre le procédé de l'invention lorsqu'un microorganisme intact est utilisé.

0,5 ml d'une suspension de spores de Streptomyces pristinaespiralis ATCC 25486 sont ajoutés en conditions stériles à 40 ml de milieu inoculum dans un erlen de 300 ml. Le milieu inoculum est constitué de 10 g/l de Corn steep ; 15 g/l de saccharose ; 10 g/l de (NH₄)₂SO₄ ; 1 g/l de K₂HPO₄ ; 3 g/l de NaCl ; 0,2 g/l de MgSO₄-7H₂O ; 1,25 g/l de CaCO₃. Le pH est ajusté à 6,9 par de la soude avant l'introduction du carbonate de calcium. L'erlen est agité pendant 46 heures 30 minutes à 27°C sur un shaker rotatif à la vitesse de 325 tr/min.

2,5 ml de la culture précédente âgée de 46 heures 30 minutes sont ajoutés stérilement à 30 ml de milieu de production dans un erlen de 300 ml. Le milieu de production est constitué de 25 g/l de farine de soja ; 7,5 g/l d'amidon ; 22,5 g/l de glucose ; 3,5 g/l de levure torula ; 0,5 g/l de sulfate de zinc ; et 6 g/l de carbonate de calcium. Le pH est ajusté à 6 avec de l'acide chlorhydrique avant l'ajout du carbonate de calcium. Les erlens sont agités pendant 24 heures à 27°C sur un shaker rotatif à la vitesse de 325 tr/min. 10 mg/l de composé (2) marqué au carbone 14 préparé selon l'exemple 1 (soit 5,2 µCi de ¹⁴C par erlen) sont ajoutés dans les erlens aux temps 0 et 17 heures 30 minutes. La réaction est stoppée au temps 24 heures par addition de 2 volumes d'un mélange de 34 % CH₃CN et 66 % tampon phosphate 0,1M pH 2,9. Après homogénéisation et centrifugation, 200 µl du surnageant sont injectés sur une colonne de nucléosil C8 5µ éluée par un mélange de 34 % CH₃CN et 66 % tampon phosphate 0,1M pH 2,9 afin de doser le composé (1) formé à partir du composé (2) radioactif. Les taux de conversion du composé (2) en composé (1) sont calculés à partir de la quantité de radioactivité retrouvée dans le composé (1), ramenée à la radioactivité introduite dans l'erlen (erlens bouchés à circulation d'air : débit 1 l/min).

Les résultats sont présentés ci-dessous:

| Temps d'ajout du composé (2) | Taux de conversion | Composé (2) résiduel % |
|---|---|---|
| 0 | 36 | 11 |
| 17 heures 30 minutes | 45 | 29 |

### Exemple 4

Le protocole décrit dans l'exemple 3 est reproduit en utilisant une suspension de spores de Streptomyces ostreogriseus ATCC 27455. Les résultats obtenus sont présentés ci-dessous :

| Temps d'ajout du composé (2) | Taux de conversion | Composé (2) résiduel % |
|---|---|---|
| 17 heures 30 minutes | 44 | 19 |

### Exemple 5

Le protocole décrit dans l'exemple 3 est reproduit en utilisant une suspension de spores de Streptomyces mitakensis ATCC 15297. Les résultats obtenus sont présentés ci-dessous :

| Temps d'ajout du composé (2) | Taux de conversion | Composé (2) résiduel % |
|---|---|---|
| 0 | 34 | 10 |
| 17 heures 30 minutes | 43 | 18 |

### Exemple 6

Le protocole décrit dans l'exemple 3 est reproduit en utilisant une suspension de spores de Streptomyces olivaceus ATCC 12019. Les résultats obtenus sont présentés ci-dessous :

| Temps d'ajout du composé (2) | Taux de conversion | Composé (2) résiduel % |
|---|---|---|
| 0 | 39 | 14 |
| 17 heures 30 minutes | 50 | 16 |

### Exemple 7

Le protocole décrit dans l'exemple 3 est reproduit en utilisant une suspension de spores de Streptomyces loïdensis ATCC 11415. Les résultats obtenus sont présentés ci-dessous :

| Temps d'ajout du composé (2) | Taux de conversion | Composé (2) résiduel % |
|---|---|---|
| 0 | 46 | 11 |
| 17 heures 30 minutes | 53 | 16 |

### Exemple 8

5 g d'un culot de centrifugation, lavé par un tampon phosphate 0,1M pH 7,2 à 10 % v/v de glycérol, d'une culture de S.olivaceus ATCC 12019 âgée de 22 heures 30 minutes obtenue dans les conditions de l'exemple 6 sont repris par 10 ml de tampon Bis-tris propane 50 mM pH 6,8.

500 µl de cette préparation sont incubés avec 3,65 µg de composé (2) marqué au carbone 14 selon l'exemple 1 (55nCi) pendant 2 heures à 27°C. 1 ml d'un mélange de 66 % de tampon phosphate 0,1M pH 2,9 et de 34 % d'acétonitrile sont ajoutés à la fin de l'incubation. Après homogénéisation et centrifugation, 200 µl du surnageant sont analysés en CLHP (exemple 3) afin de doser le composé (1) radioactif.

Le taux de conversion du composé (2) en composé (1) est de 30 %.

### Exemple 9

5 g d'un culot de centrifugation, lavé par un tampon phosphate 0,1M pH 7,2 à 10 % v/v de glycérol, d'une culture de S.loïdensis ATCC 11415 âgée de 17 heures 30 minutes obtenue dans les conditions de l'exemple 7 sont repris par 10 ml de tampon Bis-tris propane 50 mM pH 6,8.

500 µl de cette préparation sont incubés avec 3,65 µg de composé (2) marqué au carbone 14 selon l'exemple 1 (55nCi) pendant 2 heures à 27°C. 1 ml d'un mélange de 66 % de tampon phosphate 0,1M pH 2,9 et de 34 % d'acétonitrile sont ajoutés à la fin de l'incubation. Après homogénéisation et centrifugation, 200 µl du surnageant sont analysés en CLHP (exemple 3) afin de doser le composé (1) radioactif.

Le taux de conversion du composé (2) en composé (1) est de 53 %.

### Exemple 10

5 g d'un culot de centrifugation, lavé par un tampon phosphate 0,1M pH 7,2 à 10 % v/v de glycérol, d'une culture de S.pristinaespiralis âgée de 19 heures obtenue dans les conditions de l'exemple 3 sont repris par 10 ml de tampon Bis-tris propane 100 mM pH 6,8 contenant 5 mM de dithiothréitol et 0,2 mg/l de lysozyme. La suspension est incubée 30 minutes à 27°C, puis centrifugée à 30000 g pendant 30 minutes. Le surnageant constitue la préparation acellulaire.

Dans une première expérience, 207 µl de cette préparation (1 mg de protéines) sont incubés dans un volume total de 500 µl dans du tampon Bis tris-propane 50 mM pH 6,8 contenant 0,25 µmole de NADH ; 1 nmole de FMN et 3,65 µg de composé (2) marqué au carbone 14 selon l'exemple 1 (55nCi) pendant 1 heure à 27°C.

Dans une deuxième expérience, on utilise 414 µl de la préparation acellulaire (2 mg de protéines).

Les réactions sont stoppées par addition de 500 µl de CH₃CN et 500 µl d'acide chlorhydrique 0,1N. Après homogénéisation et centrifugation, 200 µl du surnageant sont injectés sur une colonne de nucléosil C8 5µ éluée par un mélange de 34 % CH₃CN et 66 % tampon phosphate 0,1M pH 2,9 afin de doser le composé (1) formé à partir du composé (2) radioactif. Les taux de conversion du composé (2) en composé (1) sont calculés comme dans l'exemple 3.

Les résultats obtenus sont présentés ci-dessous :

| Volume de suspension | Taux de conversion | Composé (2) résiduel % |
|---|---|---|
| 207 µl | 63 | 29 |
| 414 µl | 76 | 17 |

### Exemple 11

5 g d'un culot de centrifugation, lavé par un tampon phosphate 0,1M pH 7,2 à 10 % v/v de glycérol, d'une culture de S.olivaceus ATCC 12019 âgée de 21 heures obtenue dans les conditions de l'exemple 6 sont repris par 10 ml de tampon Bis-tris propane 100 mM pH 6,8 contenant 5 mM de dithiothréitol et 0,2 mg/l de lysozyme. La suspension est incubée 30 minutes à 27°C, puis centrifugée à 30000 g pendant 30 minutes. Le surnageant constitue la préparation acellulaire.

Un extrait acellulaire (22 µl de la préparation ci-dessus) contenant 0,1 mg de protéines, est incubé dans un volume total de 500 µl dans du tampon Bis-tris propane 50 mM pH 6,8 contenant 0,25 µmole de NADH ; 1 nmole de FMN et 3,65 µg de composé (2) marqué au carbone 14 selon l'exemple 1 (55nCi) pendant 1 heure à 27°C.

La réaction est stoppée par addition de 500 µl de CH₃CN et 500 µl d'acide chlorhydrique 0,1N. Après homogénéisation et centrifugation, 200 µl du surnageant sont analysés en CLHP afin de doser le composé (1) radioactif.

Le taux de conversion du composé (2) en composé (1) est de 93 %.

### Exemple 12

5 g d'un culot de centrifugation, lavé par un tampon phosphate 0,1M pH 7,2 à 10 % v/v de glycérol, d'une culture de S.loïdensis ATCC 11415 âgée de 17 heures 30 minutes obtenue dans les conditions de l'exemple 7 sont repris par 10 ml de tampon Bis-tris propane 100 mM pH 6,8 contenant 5 mM de dithiothréitol et 0,2 mg/l de lysozyme. La suspension est incubée 30 minutes à 27°C, puis centrifugée à 30000 g pendant 30 minutes. Le surnageant constitue la préparation acellulaire.

79 µl de cette préparation acellulaire, soit 0,2 mg de protéines, sont incubés dans un volume total de 500 µl dans du tampon Bis-tris propane 50 mM pH 6,8 contenant 0,25 µmole de NADH ; 1 nmole de FMN et 3,65 µg de composé (2) marqué au carbone 14 selon l'exemple 1 (55nCi) pendant 1 heure à 27°C.

La réaction est stoppée par addition de 500 µl de CH₃CN et 500 µl d'acide chlorhydrique 0,1N. Après homogénéisation et centrifugation, 200 µl du surnageant sont analysés en CLHP afin de doser le composé (1) radioactif.

Le taux de conversion du composé (2) en composé (1) est de 92 %.

## Revendications

1. Procédé de préparation d'un composé de formule (1) 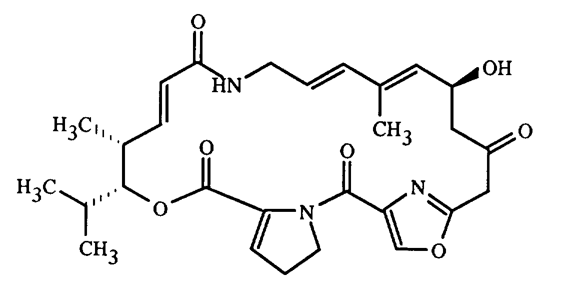 par traitement d'un composé de formule (2) 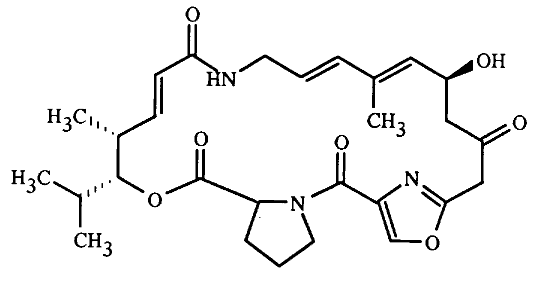 en présence d'un microorganisme ou d'une préparation acellulaire issue de ce microorganisme, procédé caractérisé en ce que :
- le microorganisme est un microorganisme producteur de streptogramines ou dérivé d'un microorganisme producteur de streptogramines capable d'oxyder la liaison 2-3 de la D-proline des macrolactones polyinsaturées du groupe A des streptogramines en déhydroproline, et,
- le traitement du composé (2) est réalisé en présence du microorganisme ou de la préparation acellulaire issue du microorganisme en phase de production optimale de l'activité enzymatique.

2. Procédé selon la revendication 1 caractérisé en ce que le microorganisme ou la préparation acellulaire issue de celui-ci est ajouté directement dans le milieu de production du composé (2).

3. Procédé selon la revendication 1 caractérisé en ce que la réaction est conduite en milieu aqueux tamponné.

4. Procédé selon l'une quelconque des revendications 1 à 3 caractérisé en ce que l'on traite le composé de formule (2) au moyen d'une préparation acellulaire.

5. Procédé selon la revendication 7 caractérisé en ce que le microorganisme est choisi parmi les souches Streptomyces pristinaespiralis, Streptomyces alborectus, Streptomyces diastaticus, Streptomyces graminofaciens, Streptomyces mitakaensis, Streptomyces loïdensis, Streptomyces olivaceus, Streptomyces ostreogriseus, Streptomyces virginiae et Micromonospora, et les dérivés de ces souches.

6. Procédé selon la revendication 4 caractérisé en ce que la réaction est conduite en présence d'un ou plusieurs cofacteurs enzymatiques.

7. Préparation acellulaire caractérisée en ce qu'elle possède une activité enzymatique capable d'oxyder la liaison 2-3 de la D-proline des macrolactones polyinsaturées du groupe A des streptogramines en déhydroproline et en ce qu'elle dérive d'un microorganisme producteur de streptogramines en phase de production optimale de l'activité enzymatique.

8. Procédé d'obtention d'une préparation acellulaire selon la revendication 9 caractérisé en ce que l'on effectue les étapes suivantes :
(a) on détermine la phase de croissance du microorganisme au cours de laquelle l'expression de l'activité enzymatique est optimale,
(b) rupture du microorganisme en phase de production optimale de l'activité enzymatique, et optionnellement,
(c) élimination des débris cellulaires.

9. Procédé selon la revendication 8 caractérisé en ce que la rupture des microorganismes est obtenue par traitement physique, chimique ou enzymatique.

10. Procédé selon les revendications 8 ou 9 caractérisé en ce que la rupture est réalisée en milieu aqueux tamponné.

## Patentansprüche

1. Verfahren zur Herstellung einer Verbindung der Formel (1) 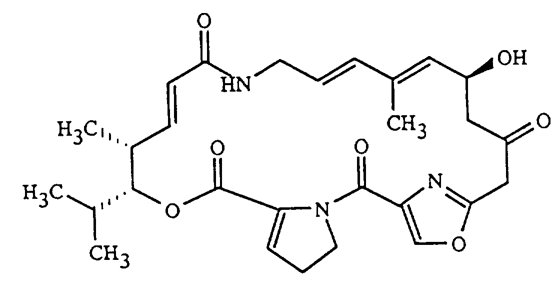 wobei man eine Verbindung der Formel (2) 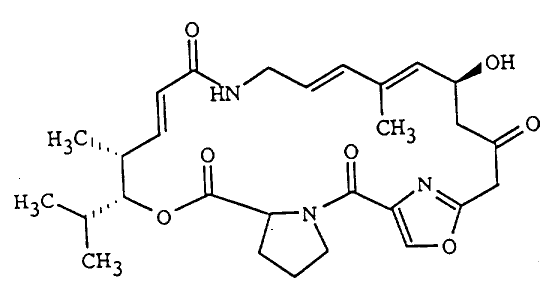 in Gegenwart eine Mikroorganismus oder eines zellfreien Präparats dieses Mikroorganismus behandelt, dadurch **gekennzeichnet**, daß
- der Mikroorganismus ein Mikroorganismus ist, der Streptogramine produziert, oder von einem Mikroorganismus, der Streptogramine produziert, abgeleitet ist, und die 2-3-Bindung von D-Prolin der mehrfach ungesättigten Makrolaktone der Gruppe A der Streptogramine in Dehydroprolin oxidieren kann, und
- man die Behandlung der Verbindung (2) in Gegenwart des Mikroorganismus oder des aus dem Mikroorganismus hervorgegangenen zellfreien Präparats in der Phase der optimalen Produktion der Enzymaktivität durchführt.

2. Verfahren nach Anspruch 1, dadurch **gekennzeichnet**, daß man den Mikroorganismus oder das daraus hervorgegangene zellfreie Präparat direkt dem Medium zur Bildung der Verbindung (2) zusetzt.

3. Verfahren nach Anspruch 1, dadurch **gekennzeichnet**, daß man die Reaktion in einem wäßrigen gepufferten Medium durchführt.

4. Verfahren nach einem der Ansprüche 1 bis 3, dadurch **gekennzeichnet**, daß man die Verbindung der Formel (2) mit einem zellfreien Präparat behandelt.

5. Verfahren nach Anspruch 1, dadurch **gekennzeichnet**, daß man den Mikroorganismus aus den Stämmen Streptomyces pristinaespiralis, Streptomyces alborectus, Streptomyces diastaticus, Streptomyces graminofaciens, Streptomyces mitakaensis, Streptomyces loidensis, Streptomyces olivaceus, Streptomyces ostreogriseus, Streptomyces virginiae und Micromonospora und Derivaten dieser Stämme auswählt.

6. Verfahren nach Anspruch 4, dadurch **gekennzeichnet**, daß man die Reaktion in Gegenwart eines oder mehrere Kofaktoren des Enzyms durchführt.

7. Zellfreies Präparat, dadurch **gekennzeichnet**, daß es eine Enzymaktivität besitzt, die die 2-3-Bindung von D-Prolin der mehrfach ungesättigten Makrolaktone der Gruppe A der Streptogramine in Dehydroprolin oxidieren kann, und daß es von einem Mikroorganismus abgeleitet ist, der Streptogramine in der Phase der optimalen Produktion der Enzymaktivität produziert.

8. Verfahren zur Gewinnung eines zellfreien Präparats nach Anspruch 7, dadurch **gekennzeichnet**, daß man die folgenden Stufen durchführt:
(a) man bestimmt die Wachstumsphase des Mikroorganismus, im Verlauf derer die Expression der Enzymaktivität optimal ist,
(b) man bricht dem Mikroorganismus in der Phase der optimalen Produktion der Enzymaktivität auf, und
(c) man beseitigt gegebenenfalls die Zelltrümmer.

9. Verfahren nach Anspruch 8, dadurch **gekennzeichnet**, daß man die Mikroorganismen durch physikalische, chemische oder enzymatische Behandlung aufbricht.

10. Verfahren nach den Ansprüchen 8 oder 9, dadurch **gekennzeichnet**, daß man in wäßrigem, gepuffertem Medium aufbricht.

## Claims

1. Process for preparing a compound of formula (1) 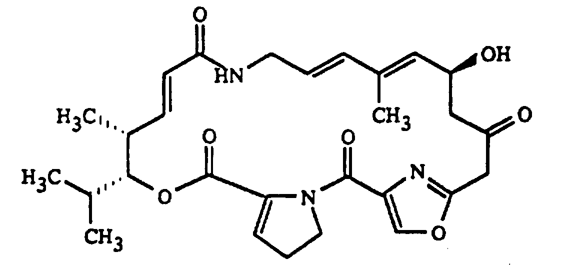 by treating a compound of formula (2) 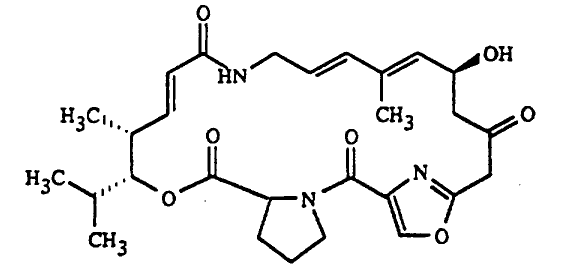 in the presence of a microorganism or an acellular preparation derived from this microorganism, the process being characterised in that
- the microorganism is a streptogramin-producing microorganism or one derived from a streptogramin-producing microorganism capable of oxidising the 2-3 bond of the D-proline of the polyunsaturated macrolactones of the group A streptogramins to dehydroproline, and
- the compound (2) is treated in the presence of the microorganism or of the acellular preparation derived from the microorganism at the phase of optimal production of the enzymatic activity.

2. Process according to Claim 1, characterised in that the microorganism or the acellular preparation derived from it is added directly to the production medium of the compound (2).

3. Process according to Claim 1, characterised in that the reaction is carried out in a buffered aqueous medium.

4. Process according to any one of Claims 1 to 3, characterised in that the compound of formula (2) is treated by means of an acellular preparation.

5. Process according to Claim 1, characterised in that the microorganism is chosen from the strains Streptomyces pristinaespiralis, Streptomyces alborectus, Streptomyces diastaticus, Streptomyces graminofaciens, Streptomyces mitakaensis, Streptomyces loïdensis, Streptomyces olivaceus, Streptomyces ostreogriseus, Streptomyces virginiae and Micromonospora, and the derivatives of these strains.

6. Process according to Claim 4, characterised in that the reaction is carried out in the presence of one or more enzymatic cofactors.

7. Acellular preparation characterised in that it possesses an enzymatic activity capable of oxidising the 2-3 bond of the D-proline of the polyunsaturated macrolactones of the group A streptogramins to dehydroproline, and in that it is derived from a streptogramin-producing microorganism at the phase of optimal production of the enzymatic activity.

8. Process for producing an acellular preparation according to Claim 7, characterised in that the following steps are carried out:
(a) determination of the growth phase of the microorganism during which the expression of enzymatic activity is optimal,
(b) disruption of the microorganism at the phase of optimal production of the enzymatic activity, and optionally,
(c) removal of the cellular debris.

9. Process according to Claim 8, characterised in that the disruption of the microorganisms is obtained by physical, chemical or enzymatic treatment.

10. Process according to Claims 8 or 9, characterised in that the disruption is carried out in a buffered aqueous medium.
